# FASCICULE DE BREVET EUROPEEN

(11) **EP 1 646 373 B1**
(45) Date de publication et mention de la délivrance du brevet: **24.09.2008**
(21) Numéro de dépôt: 04767690.3
(22) Date de dépôt: 16.07.2004
(51) Int. Cl.: A61K 31/14, A61K 9/20, A61P 9/14

(54) **COMPOSITION PHARMACEUTIQUE ORODISPERSIBLE D'UN COMPOSE ANTITHROMBOTIQUE**
ORODISPERSIBLE PHARMAZEUTISCHE ZUSAMMENSETZUNG EINER ANTITHROMBOTISCHEN VERBINDUNG
ORODISPERSIBLE PHARMACEUTICAL COMPOSITION OF AN ANTITHROMBOTIC COMPOUND

(30) Priorité: 18.07.2003 FR 0308780
(43) Date de publication de la demande: 19.04.2006
(73) Titulaire: Les Laboratoires Servier, 92415 Courbevoie Cédex (FR)
(72) Inventeur: WUTHRICH, Patrick, F-45000 Orléans (FR); ROLLAND, Hervé, F-45160 Olivet (FR); JULIEN, Marc, F-45110 Sigloy (FR)
(86) Numéro de dépôt international: PCT/FR2004/001866
(87) Numéro de publication internationale: WO 2005/009428

(56) Documents cités:
- US-A- 5 194 614
- US-A- 5 472 979
- US-A1- 2002 035 248
- BAYES M ET AL: "Gateways to clinical trials." METHODS AND FINDINGS IN EXPERIMENTAL AND CLINICAL PHARMACOLOGY, vol. 25, no. 6, juillet 2003 (2003-07), pages 483-506, XP008028377 ISSN: 0379-0355 (ISSN print)
- KAKKOS STAVROS K ET AL: "S-18886 Servier." CURRENT OPINION IN INVESTIGATIONAL DRUGS (LONDON, ENGLAND: 2000) ENGLAND SEP 2002, vol. 3, no. 9, septembre 2002 (2002-09), pages 1324-1327, XP008028385 ISSN: 1472-4472
- BELHASSEN LAURENT ET AL: "Improved endothelial function by the thromboxane A2 receptor antagonist S 18886 in patients with coronary artery disease treated with aspirin." JOURNAL OF THE AMERICAN COLLEGE OF CARDIOLOGY. UNITED STATES 2 APR 2003, vol. 41, no. 7, 2 avril 2003 (2003-04-02), pages 1198-1204, XP002272663 ISSN: 0735-1097

## Description

La présente invention a pour objet une forme pharmaceutique orodispersible solide pour l'administration par vo0e orale ou buccale d'un composé antithrombotique ou d'un de ses sels pharmaceutiquement acceptables.

Ce composé antithrombotique, ci-après dénommé composé A, décrit dans le brevet EP 648741 est le composé de formule (I) :

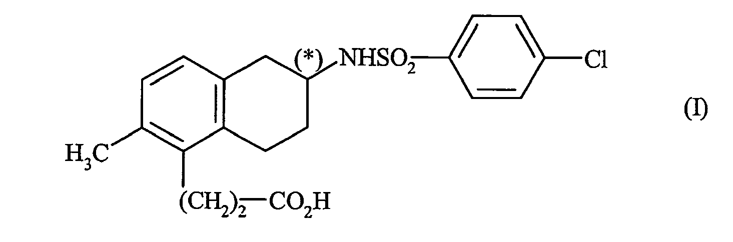

Le composé A peut être administré par voie orale sous forme de comprimés à avaler avec un demi-verre d'eau.

Les doses du composé A utilisées par voie orale ou parentérale pour obtenir l'effet thérapeutique vont généralement de 10 mg à 30 mg par prise, une à plusieurs fois par jour, sous la forme de comprimé à libération immédiate.

De nombreuses personnes ont des difficultés pour avaler les comprimés conventionnels souvent de taille non négligeable. Les problèmes liés à l'ingestion de médicaments (étouffement, suffocation par obstruction de la gorge) sont souvent à l'origine d'un mauvais respect des posologies, voire d'un arrêt du traitement.

Les compositions pharmaceutiques de la présente invention permettent non seulement de remédier aux inconvénients connus de la forme comprimé à avaler mais également de proposer un service médical rendu supérieur permettant notamment l'amélioration de la qualité de vie des patients.

La composition pharmaceutique orodispersible du composé A présente l'avantage d'une obtention rapide de taux plasmatiques élevés en principe actif. Elle permet de plus grâce à une désagrégation rapide de limiter les variabilités d'absorption qui peuvent être causées par différents facteurs.

La composition pharmaceutique orodispersible selon l'invention présente la particularité de ne nécessiter ni eau ni mastication au cours de son administration. Elle se désagrège très rapidement dans la bouche, de préférence en moins de trois minutes et de manière encore plus préférentielle en moins d'une minute.

De nombreuses formes à dissolution rapide sont décrites dans l'art antérieur. De manière générale, les technologies décrites précédemment ont en commun l'utilisation d'un agent désintégrant comme le Kollidon^{®} CL (polyvinylpyrrolidone réticulée), l'EXPLOTAB^{®} (fécule carboxyméthylée), l'AC DISOL^{®} (carboxyméthylcellulose sodique réticulée).

Cet agent de désintégration est indispensable dans la formulation des comprimés orodispersibles et doit être utilisé conjointement avec un excipient de compression directe. Les difficultés rencontrées pour la fabrication de tels comprimés résident dans le fait qu'il est très difficile d'obtenir des comprimés présentant des caractéristiques physiques constantes et reproductibles et compatibles avec les contraintes de manipulation classiques des comprimés.

En effet, les mélanges classiquement utilisés conduisent à des comprimés de dureté très élevée totalement inadaptée à une désagrégation rapide dans la cavité buccale.

D'autres formes orodispersibles sont réalisables par l'utilisation de la lyophilisation aboutissant à l'obtention de formes solides très poreuses dénommées "lyophilisat oral". Ces formes nécessitent l'utilisation d'un procédé industriel très spécifique, compliqué et long de mise en oeuvre, donnant une forme médicamenteuse à prix de revient élevé.

La présente invention permet de remédier à ces inconvénients. Elle concerne une forme solide orodispersible du composé A éventuellement sous forme d'isomère optique ou d'un de ses sels pharmaceutiquement acceptables contenant un excipient simple, d'origine naturelle permettant la désagrégation rapide, présentant une neutralité gustative et de texture agréable. Cet excipient joue le rôle à la fois de liant et de désintégrant. Il permet d'obtenir une formulation du composé A simple, ayant une excellente aptitude à la compression directe conduisant à des comprimés de faible friabilité et de dureté compatible avec les techniques classiques de manipulation.

Plus particulièrement, l'invention concerne une composition pharmaceutique solide orodispersible du composé A ou d'un de ses sels pharmaceutiquement acceptables caractérisée en ce qu'elle contient :
- le composé A ou un de ses sels pharmaceutiquement acceptables,
- et des granules consistant en lactose et amidon coséchés.

Le composé A possède préférentiellement la configuration absolue (R).

De manière préférentielle, le composé A se présente sous forme de sel de sodium.

La composition selon l'invention peut également contenir, pour des raisons de fabrication, un ou plusieurs lubrifiants et un agent d'écoulement, et pour des raisons de masquage de goût ou d'amertume, des arômes et des édulcorants, classiquement utilisés.

Pour améliorer le masquage d'amertume du composé A, celui-ci pourra éventuellement être associé à des excipients comme les cyclodextrines, ou enrobé avec des excipients par l'utilisation de technologies connues de l'Homme de l'Art comme par exemple l'enrobage en lit d'air fluidisé, l'atomisation, la coacervation, le prilling, le spray congealing.

L'invention a également pour objet l'utilisation de granules consistant en lactose et amidon coséchés pour la préparation de compositions pharmaceutiques solides orodispersibles du composé A.

On entend par le terme "orodispersible" des compositions pharmaceutiques solides qui se délitent dans la cavité buccale en moins de 3 minutes, et de préférence en moins d'une minute.

Lesdits granules compris dans les compositions pharmaceutiques solides selon l'invention correspondent aux compositions décrites dans la demande de brevet EP 00/402159.8. Ces granules sont caractérisés par une structure sphérique et une comprimabilité avantageuse et sont commercialisés sous l'appellation STARLAC^{®}.

Les propriétés désintégrantes desdits granules sont connues pour des comprimés placés dans des volumes de liquides importants, sous agitation. Il est particulièrement surprenant que de tels granules employés pour la fabrication de formes orodispersibles puissent donner des résultats particulièrement satisfaisants en terme de désagrégation en bouche, et ce pour deux raisons.

La première est basée sur le constat que les excipients les moins solubles dans l'eau sont les plus appropriés à la formulation de comprimés orodispersibles (la solubilisation, entraînant une augmentation de viscosité de l'eau, est un frein à sa pénétration dans les comprimés). Or lesdits granules comprennent une fraction importante de lactose très soluble dans l'eau. De plus, l'amidon compris dans lesdits granules n'est pas un agent "super désintégrant" tel qu'utilisé et décrit dans les formes orodispersibles de l'art antérieur.
La deuxième est basée sur le constat que les propriétés de désintégration d'un excipient (utilisé dans un comprimé) évaluées dans l'eau par les méthodes conventionnelles ne sont pas extrapolables au comportement du même comprimé in vivo, dans la salive. En effet, les vitesses de désintégration dans l'eau sont mesurées (selon la Pharmacopée Européenne) dans une quantité d'eau suffisamment importante pour ne pas atteindre la saturation en terme de solubilisation, alors que in vivo, de par le faible volume de salive, les excipients sont à saturation. De plus, l'agitation à laquelle sont soumis les comprimés lors du test usuel ne reflète pas la désagrégation en bouche. La Demanderesse a ainsi constaté lors d'essais comparatifs que certains excipients connus comme bons désintégrants n'étaient pas adaptés à la préparation de formes orodispersibles. Inversement, certains excipients se désintégrant moyennement dans l'eau peuvent présenter des propriétés avantageuses in vivo.

La Demanderesse a alors trouvé que lesdits granules conféraient de façon surprenante aux comprimés de très bonnes aptitudes à se désagréger en bouche, et ce pour une large gamme de duretés de comprimés, tout en conservant une friabilité faible ce qui est particulièrement remarquable. En effet, la plupart des formes orodispersibles de l'art antérieur qui se délitent rapidement dans la bouche sont très friables, ce qui se traduit par la nécessité d'utiliser un conditionnement spécifique et par des risques de désagrégation du comprimé dès qu'il est manipulé et ôté de son emballage.

Il est particulièrement remarquable que les critères d'orodispersibilité et de friabilité faible précités soient respectés pour une large gamme de dureté de comprimés, c'est-à-dire pour des comprimés présentant une dureté comprise entre 15 et 30 Newtons.

Les compositions pharmaceutiques selon l'invention sont préférentiellement caractérisées en ce qu'elles contiennent, par rapport au poids total du comprimé :
- de 2,5 % à 20 % en poids du composé A ou d'un de ses sels pharmaceutiquement acceptables, préférentiellement de 5 % à 10 %,
- de 75 % à 95 % en poids de STARLAC^{®}.

Elles contiendront éventuellement de 0,1 % à 3 % en poids d'agents lubrifiants comme le stéarate de magnésium, préférentiellement de 0,5 % à 1,5 % et de 0,1 % à 3 % en poids d'un agent d'écoulement comme la silice colloïdale, préférentiellement de 0,5 % à 1,5 %.

Les exemples suivants illustrent l'invention mais ne la limitent en aucune façon :

Les comprimés orodispersibles ont été réalisés avec l'isomère (R) du composé A sous forme de sel de sodium.

### EXEMPLE 1 :

### Formulation : Comprimé terminé à 100 mg

| ***Constituants*** | ***Quantité (mg)*** |
|---|---|
| Composé A, sel de sodium | 10* |
| Starlac^{®} | 88,25 |
| Stéarate de magnésium | 1 |
| Silice colloïdale anhydre | 0,25 |
| Aspartam | 0,25 |
| Acesulfame K | 0,25 |

| | |
|---|---|
| * exprimé en composé A, forme base | |

### EXEMPLE 2 :

### Formulation : Comprimé terminé à 300 mg

| ***Constituants*** | ***Quantité (mg)*** |
|---|---|
| Composé A, sel de sodium | 30* |
| Starlac^{®} | 264,75 |
| Stéarate de magnésium | 3 |
| Silice colloïdale anhydre | 0,75 |
| Aspartam | 0,75 |
| Acesulfame K | 0,75 |

| | |
|---|---|
| * exprimé en composé A, forme base | |

Les comprimés sont préparés par mélange des constituants suivi d'une compression directe. Les duretés des comprimés des exemples 1 et 2 sont environ égales à 15 Newtons et 30 Newtons respectivement.

Afin d'évaluer le temps de désagrégation en bouche, les comprimés orodispersibles du composé A décrits dans les exemples 1 et 2 ont été placés dans la bouche. Lors de ces tests, il s'est avéré que pour chacune des formulations testées le temps de désagrégation dans la bouche était inférieur à 1 minute.

## Revendications

1. Composition pharmaceutique solide orodispersible du composé A de formule (I) éventuellement sous forme d'isomère optique ou d'un de ses sels pharmaceutiquement acceptables : 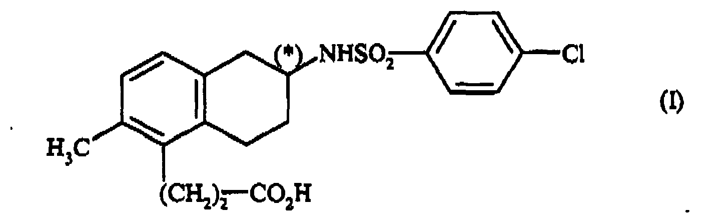 **caractérisée en ce qu'**elle comprend :
- le composé A, ou un de ses sels pharmaceutiquement acceptables,
- des granules consistant en lactose et amidon coséchés.

2. Composition pharmaceutique selon la revendication 1 **caractérisée en ce que** le composé A est sous la forme d'un isomère optique de configuration (R).

3. Composition pharmaceutique selon l'une quelconque des revendications 1 ou 2 **caractérisée en ce qu'**elle comprend, par rapport au poids total de la composition :
- de 2,5 % à 20 % en poids du composé A ou d'un de ses sels pharmaceutiquement acceptables,
- de 75 % à 95 % en poids de granules consistant en lactose et amidon coséchés.

4. Composition pharmaceutique selon la revendication 3 **caractérisée en ce qu'**elle comprend de 5 % à 10 % en poids du composé A ou d'un de ses sels pharmaceutiquement acceptables.

5. Composition pharmaceutique selon l'une quelconque des revendications 1 à 4 **caractérisée en ce que** le composé A est sous la forme d'un sel de sodium.

6. Composition pharmaceutique selon la revendication 1 **caractérisée en ce qu'**elle comprend également un ou plusieurs arômes, et édulcorants.

7. Composition pharmaceutique selon la revendication 1 **caractérisée en ce qu'**elle comprend également un ou plusieurs lubrifiants, et un agent d'écoulement.

8. Composition pharmaceutique selon l'une quelconque des revendications 1 à 7 **caractérisée en ce qu'**elle se présente sous forme de comprimé.

9. Comprimé selon la revendication 8 **caractérisé en ce qu'**il est obtenu par compression directe.

10. Comprimé selon la revendication 9 **caractérisé en ce que** sa dureté est comprise entre 15 et 30 Newtons.

11. Utilisation de granules consistant en lactose et amidon coséchés dans la fabrication des compositions solides orodispersibles du composé A se délitant en bouche en moins de trois minutes et de préférence en moins d'une minute, pour l'obtention d'un médicament destiné à une administration orale ou buccale.

12. Composition pharmaceutique solide orodispersible du composé A selon la revendication 1, ou d'un de ses sels pharmaceutiquement acceptables, pour l'utilisation comme médicament antithrombotique.

## Claims

1. Solid orodispersible pharmaceutical composition of compound A of formula (I), optionally in the form of an optical isomer, or a pharmaceutically acceptable salt thereof : 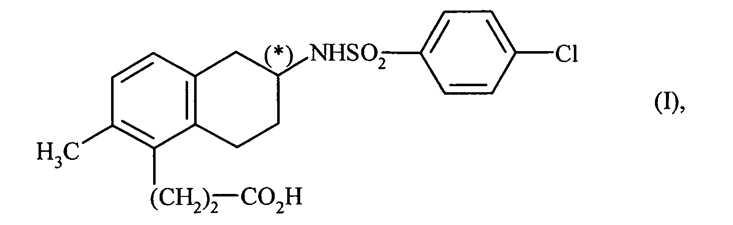 **characterised in that** it comprises :
- compound A or a pharmaceutically acceptable salt thereof,
- granules consisting of co-dried lactose and starch.

2. Pharmaceutical composition according to claim 1, **characterised in that** compound A is in the form of an optical isomer of configuration (R).

3. Pharmaceutical composition according to either claim 1 or claim 2, **characterised in that** it comprises, in relation to the total weight of the composition :
- from 2.5 % to 20 % by weight of compound A or a pharmaceutically acceptable salt thereof,
- from 75 % to 95 % by weight of granules consisting of co-dried lactose and starch.

4. Pharmaceutical composition according to claim 3, **characterised in that** it comprises from 5 % to 10 % by weight of compound A or a pharmaceutically acceptable salt thereof.

5. Pharmaceutical composition according to any one of claims 1 to 4, **characterised in that** compound A is in the form of a sodium salt.

6. Pharmaceutical composition according to claim 1, **characterised in that** it also comprises one or more flavourings, and sweeteners.

7. Pharmaceutical composition according to claim 1, **characterised in that** it also comprises one or more lubricants and a flow agent.

8. Pharmaceutical composition according to any one of claims 1 to 7, **characterised in that** it is in the form of a tablet.

9. Tablet according to claim 8, **characterised in that** it is obtained by direct compression.

10. Tablet according to claim 9, **characterised in that** its hardness is from 15 to 30 Newtons.

11. Use of granules consisting of co-dried lactose and starch in the manufacture of solid orodispersible compositions of compound A, which disintegrate in the mouth in less than three minutes, preferably less than one minute, for obtaining a medicament intended for oral or buccal administration.

12. Solid orodispersible pharmaceutical composition of compound A according to claim 1, or a pharmaceutically acceptable salt thereof, for use as an antithrombotic medicament.

## Patentansprüche

1. Feste, im Mundraum dispergierbare pharmazeutische Zubereitung der Verbindung (A) der Formel (I), gegebenenfalls in Form des optischen Isomeren oder eines seiner pharmazeutisch annehmbaren Salze: 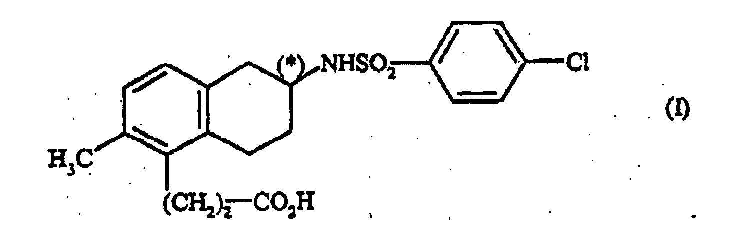 **dadurch gekennzeichnet, dass** sie:
- die Verbindung A oder eines ihrer pharmazeutisch annehmbaren Salze und
- aus gemeinsam getrockneter Lactose und Stärke bestehende Körnchen umfasst.

2. Pharmazeutische Zubereitung nach Anspruch 1, **dadurch gekennzeichnet, dass** die Verbindung (A) in Form des optischen Isomeren in der Konfiguration (R) vorliegt.

3. Pharmazeutische Zubereitung nach einem der Ansprüche 1 oder 2, **dadurch gekennzeichnet, dass** sie, bezogen auf das Gesamtgewicht der Zubereitung:
- 2,5 bis 20 Gew.-% der Verbindung A oder eines ihrer pharmazeutisch annehmbaren Salze und
- 75 bis 95 Gew.-% der aus gemeinsam getrockneter Lactose und Stärke bestehenden Körnchen enthält.

4. Pharmazeutische Zubereitung nach Anspruch 3, **dadurch gekennzeichnet, dass** sie 5 bis 10 Gew.-% der Verbindung A oder eines ihrer pharmazeutisch annehmbaren Salze enthält.

5. Pharmazeutische Zubereitung nach einem der Ansprüche 1 bis 4, **dadurch gekennzeichnet, dass** die Verbindung A in Form des Natriumsalzes vorliegt.

6. Pharmazeutische Zubereitung nach Anspruch 1, **dadurch gekennzeichnet, dass** sie zusätzlich ein oder mehrere Aromastoffe und Süßungsmittel enthält.

7. Pharmazeutische Zubereitung nach Anspruch 1, **dadurch gekennzeichnet, dass** sie zusätzlich ein oder mehrere Gleitmittel und ein Fließverbesserungsmittel enthält.

8. Pharmazeutische Zubereitung nach einem der Ansprüche 1 bis 7, **dadurch gekennzeichnet, dass** sie in Form einer Tablette vorliegt.

9. Tablette nach Anspruch 8, **dadurch gekennzeichnet, dass** sie durch direktes Verpressen erhalten worden ist.

10. Tablette nach Anspruch 9, **dadurch gekennzeichnet, dass** ihre Härte zwischen 15 und 30 Newton liegt.

11. Verwendung von Körnchen, die aus gemeinsam getrockneter Lactose und Stärke bestehen, bei der Herstellung von festen, im Mundraum dispergierbaren Zubereitungen der Verbindung A, die sich in weniger als drei Minuten, vorzugsweise in weniger als einer Minute im Mundraum zersetzen, zur Herstellung eines Arzneimittels, das für die orale oder bukkale Verabreichung bestimmt ist.

12. Feste, im Mundraum dispergierbare pharmazeutische Zubereitung der Verbindung A oder eines ihrer pharmazeutisch annehmbaren Salze nach Anspruch 1 zur Verwendung als antithrombotisches Arzneimittel.
